# EUROPEAN PATENT SPECIFICATION

(11) **EP 1 985 329 B1**
(45) Date of publication and mention of the grant of the patent: **02.06.2010**
(21) Application number: 07008444.7
(22) Date of filing: 25.04.2007
(51) Int. Cl.: A61B 17/29, A61B 1/00

(54) **Tubular member and endoscopic instrument**
Rohrförmiges Element und endoskopisches Instrument
Élément tubulaire et instrument endoscopique

(43) Date of publication of application: 29.10.2008
(73) Proprietor: OLYMPUS MEDICAL SYSTEMS CORP., Tokyo (JP)
(72) Inventor: Kimura, Koh, 43-2, Hatagaya 2-chome Shibuya-ku, Tokyo (JP)
(74) Representative: von Hellfeld, Axel

(56) References cited:
- EP-A1- 0 738 501
- EP-A1- 1 325 709
- WO-A-97/41776
- WO-A-98/01080
- WO-A-2007/002179
- US-A- 5 137 013
- US-B2- 6 717 092

## Description

### BACKGROUND OF THE INVENTION

### Field of the Invention

The present invention relates to a tubular member which is used with an endoscope in an endoscopic surgery, and also relates to an endoscopic instrument having the tubular member.

### Description of the Related Art

In endoscopic surgery, an endoscopic instrument of which a treatment portion such as a forceps or the like is disposed on a distal end of a sheath is used. For example, in Japanese Unexamined Patent Application, First Publication No. 2000-52029 , a connecting member which connects the treatment portion of the endoscopic instrument with the distal end of the sheath is disclosed.

Depending on the type of surgery, in order to change the flexibility of the sheath on the way of the sheath, it is performed that one sheath is connected to another sheath which differs from the one sheath. For example, in Japanese Utility Model Application, First Publication No. H05-15914 , an endoscopic instrument which includes the different sheaths connected to each other is disclosed.

WO2007/002179 discloses a medical device connector for connecting the shafts of two or more catheters. A larger end of the connector receives the distal and of the first catheter, and a smaller end of the connector receives the proximal end of the second catheter. The catheter shafts are secured to the connector by overmolding, heat staking, adhesive, crimping, welding, or insertion prongs. The catheter is shaped to house the two catheters having two different diameters.

US 5,137,013 describes a joining structure for a coil wound metal wire flexible tube and another member. The other member comprises a main joint joining the end of the outer peripheral surface of the flexible tube to the end of the other member. The flexible tube is inserted and engaged into the other member, and then the two members are laser welded together by melting the area where the flexible coil is inserted into the other member. Subsequently, adjacent turns of the coil near the welded region are spot welded together in order to strengthen areas weakened from the thermal influence of the laser welding.

US 6,717,092 discloses a connection pipe for connecting two different catheters by inserting the first and second coils into the pipe from opposite ends thereof, thereby exposing both ends of the connection pipe. The end portions of the pipe are melted to the corresponding portions of the catheters. In another embodiment, through holes are formed on the pipe and are submitted to an arc column. The arc column melts the portion of the catheters exposed through the through holes, fixing the catheters to the pipe.

WO98/01080 discloses two connecting portions on each of two coils which couple together to join the two coils. The connecting portions are crimped, soldered, or welded to the portions of the coils inserted into the connecting portions.

EP 1 325 709 discloses a flexible surgical instrument having a rotatable distal attachment. The instrument is rotatably coupled to the sheath via a bushing in a housing. The housing is attached to the distal end of the coil by crimping welding, or soldering.

EP 0 738 501 discloses an endoscope in which a forward-end member is coupled with a sheath via a engaging member on the sheath having an increased diameter and a proximal portion of the forward-end portion having a reduced diameter, thereby rotatably fixing the forward-end member and the sheath.

WO 97/14776 discloses a cylindrical connector having an area of reduced diameter adapted to received inside a flexible coil, wherein the coil is thereafter bonded to the area of reduced diameter.

### SUMMARY OF THE INVENTION

A tubular member of the present invention is defined in independent claim 1.

### BRIEF DESCRIPTION OF THE DRAWINGS

FIG. 1 is a view showing the construction of an endoscopic instrument of the first embodiment.
FIG. 2 is a partial cross-sectional view showing the construction of a tubular member, and showing a state in which adjacent sheaths are connected to each other.
FIG. 3 is a side view showing a state in which the sheath and a tubular member are connected when the sheath is bent.
FIG. 4 is a partial cross-sectional view showing a first variant relative to the tubular member and a method for connecting the sheaths of this embodiment.
FIG. 5 is a side view showing a state in which the sheath and a tubular member are connected when the sheath is bent.
FIG. 6 is a view showing the construction of an endoscopic instrument of the second embodiment.
FIG 7 is a partial cross-sectional view showing the construction of a tubular member, and showing a state in which a treatment portion and a sheath are connected to each other.
FIG. 8 is a partial cross-sectional view showing a first variant relative to the tubular member and a method for connecting the sheaths of this embodiment.
FIG. 9 is a partial cross-sectional view showing a second variant relative to the tubular member and a method for connecting the sheaths of this embodiment.
FIG. 10 is a partial cross-sectional view showing a third variant relative to the tubular member and a method for connecting the sheaths of this embodiment.
FIG. 11 is a partial cross-sectional view showing a fourth variant relative to the tubular member and a method for connecting the sheaths of this embodiment.
FIG. 12 is a partial cross-sectional view showing a fifth variant relative to the tubular member and a method for connecting the sheaths of this embodiment.

### DESCRIPTION OF THE PREFERRED EMBODIMENTS

### (First embodiment)

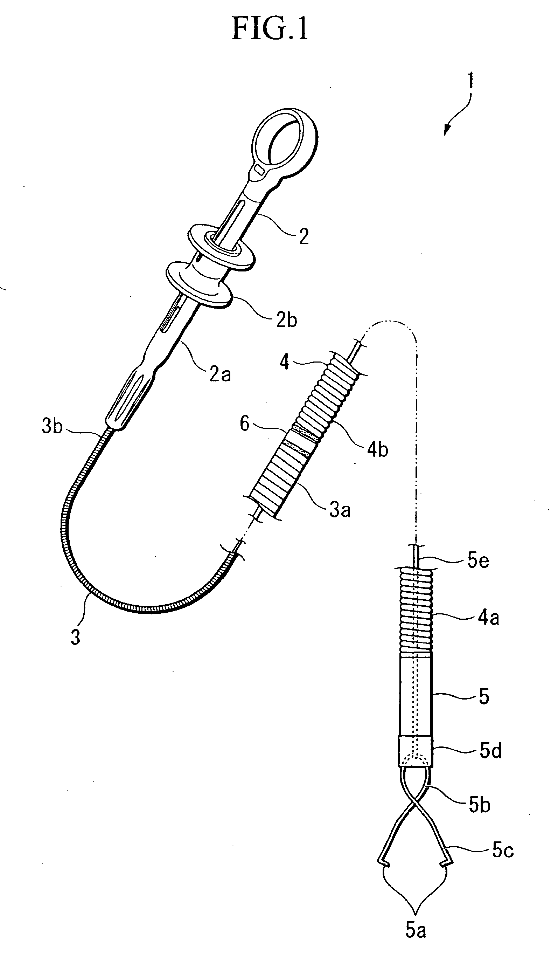

An endoscopic instrument of a first embodiment is shown in FIG. 1. As shown in FIG. 1, the endoscopic instrument 1 includes: an operation portion 2 which is operated by an operator; a first sheath 3 which extends from the operation portion 2; a second sheath 4 which extends from a distal end 3a of the first sheath 3; a treatment portion 5 which is disposed on a distal end 4a of the second sheath 4; and a tubular member 6 which connects the first sheath 3 and the second sheath 4.

The first sheath 3 and the second sheath 4 are formed like a tube, external diameters thereof are approximately 2 to 4 millimeters, and internal diameters thereof are approximately 0.5 to 3 millimeters. The first sheath 3 and the second sheath 4 are made of metal, and are formed in a coil-shape. Thus, the first sheath 3 and the second sheath 4 have flexibility. The first sheath 3 is a flat coil sheath which is formed by winding a plate-shaped wire rod like a coil, and the second sheath 4 is a round coil sheath which is formed by winding a round wire rod like a coil. Since the shape and thickness of the wire rod of the first sheath 3 differ from that of the second sheath 4, the bending flexibility is different relatively. Therefore, the second sheath 4 is able to bend easily.

The treatment portion 5 is a clip, including: a clip main body 5c; a cover 5d which is formed like a tube; and an operating wire 5e. Two hook portions 5a are formed on each end of the clip main body 5c, and the clip main body 5c is bent so that a ring portion 5b is formed on a proximal end of the treatment portion 5c. The operating wire 5e is inserted into the cover 5d, and is connected to the ring portion 5b of the clip main body 5c. The diameter of the ring portion 5b is set so as to be larger than the internal diameter of the cover 5d. The cover 5d is able to be attached to the second sheath 4 so as to insert the second sheath 4 into a proximal end of the cover 5d. The operating wire 5e is inserted into the first sheath 3 and the second sheath 4, and is connected to the operation portion 2 which is disposed on a proximal end 3b of the first sheath 3.

As shown in FIG.1, the operation portion 2 includes a holding portion 2a and a slider 2b which is able to move along the holding portion 2a in an axis direction of the holding portion 2a. The operating wire 5e is connected to the slider 2b. When the slider 2b is pulled toward a proximal end of the holding portion 2a, the ring portion 5b of the treatment portion 5 is drawn into the cover 5d, thus the ring portion 5b is contracted corresponding to the internal diameter of the cover 5b. Therefore, the hook portions 5a arranged at both ends of the clip main body 5c are closed. As a result, it is possible to nip a predetermined part with the hook portions 5a.

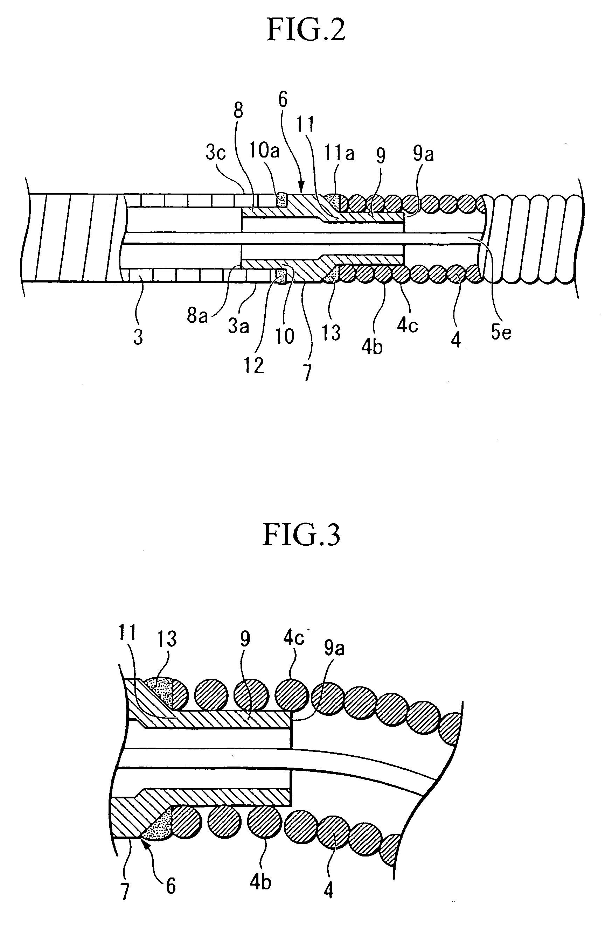

As shown in FIG. 2, the tubular member 6 is formed in a tube-shape approximately, and is made of metal. The tubular member 6 includes: a main body portion 7 which is disposed between the first sheath 3 and the second sheath 4 so that an axis of the main body portion 7 matches that of the first sheath 3 and the second sheath 4; a first sheath receiving portion 8 and a first sheath connecting portion 10 which are disposed on a proximal end portion of the main body portion 7; and a second sheath receiving portion 9 and a second sheath connecting portion 11 which are disposed on a distal end portion of the main body portion 7.

The first sheath receiving portion 8 is engaged inside of the first sheath 3, and the first sheath connecting portion 10 is disposed between the main body portion 7 and the first sheath receiving portion 8. The second sheath receiving portion 9 is engaged inside of the second sheath 4, and the second sheath connecting portion 11 is disposed between the main body portion 7 and the second sheath receiving portion 9. The external diameter of the first sheath receiving portion 8 is substantially equal to the internal diameter of the first sheath 3, and is set so as to be removably attached in the axial direction with ease. Similarly, the external diameter of the second sheath receiving portion 9 is equal to the internal diameter of the second sheath 4, and is set so as to be removably attached in the axial direction with ease.

A step portion 10a which contacts the distal end 3a of the first sheath 3 engaged with the first sheath receiving portion 8 is formed on the first sheath connecting portion 10. Similarly, a step portion 11a which contacts a proximal end 4b of the second sheath 4 is formed on the second sheath connecting portion 11. The first sheath 3 and the tubular member 6 which are made of metal are heated at the first sheath connecting portion 10, and are connected to each other by forming a welding portion 12 so as to weld the first sheath 3 and the tubular member 6 in a circumferential direction using a laser beam. Similarly, the second sheath 4 and the tubular member 6 are heated at the second sheath connecting portion 11, and are connected by forming a welding portion 13 so as to weld the second sheath 4 and the tubular member 6 in a circumferential direction using a laser beam. Note that, the length in an axial direction of the first sheath receiving portion 8 and the first sheath connecting portion 10 which are engaged with the first sheath 3, and the length in an axial direction of the second sheath receiving portion 9 and the second sheath connecting portion 11 which are engaged with the second sheath 4, are approximately 1 to 5 millimeters.

Next, the action of this embodiment will be explained.

As shown in FIG. 2, the distal end 3a of the first sheath 3 is fixed to the first sheath connecting portion 10 of the tubular member 6 by the welding portion 12 so as to prevent the first sheath 3 from separating from the sheath connecting portion 10 in an axial direction. Further, the first sheath 3 is supported in a radial direction so as to oppose bending by the first sheath receiving portion 8 of the tubular member 6, which is closer to a proximal end of the tubular member 6 than the welding portion 12. Similarly, the proximal end 4b of the second sheath 4 is fixed to the second sheath connecting portion 11 of the tubular member 6 by the welding portion 13 so as to prevent the second sheath 4 from separating from the tubular member 6 in an axial direction. Further, the second sheath 4 is supported in a radial direction so as to oppose bending by the second sheath receiving portion 9 of the tubular member 6, which is closer to a proximal end than the welding portion 13.

In the endoscopic instrument 1, for example, the first sheath 3, the tubular member 6, the second sheath 4 and the treatment portion 5, which are connected as a single body as described above, are inserted into a channel of the endoscope, and used. At this time, the first sheath 3 and the second sheath 4, which have flexibility, are bent, but the tubular member 6 is not bent. Thus, between the tubular member 6 and the first sheath 3, and between the tubular member 3 and the second sheath 4, an axial force which causes separation acts, and a bending action occurs. That is, in each of the first sheath 3 and the second sheath 4, a stress caused by bending and a stress caused by separation are generated.

As shown in FIG. 3, for example, since the second sheath 4 is supported in the radial direction by the second sheath receiving portion 9, the stress caused by bending is generated at a position 4c corresponding to a distal end 9a of the second sheath receiving portion 9 of the tubular member 6. On the other hand, the stress caused by separation is generated at the welding portion 13 which fixes the proximal end 4b of the second sheath 4. At this time, the second sheath 4 is not restricted in an axial direction at the second sheath receiving portion 9, that is, the second sheath 4 is free to move along the second sheath receiving portion 9. Accordingly, the spiral wire rod forming a coil extends due to the stress, and the second sheath 4 gets longer along the second sheath receiving portion 9. As a result, the stress caused by bending is dispersed, and is diffused.

Similarly, in the first sheath 3, as shown in FIG. 2, the stress caused by bending is generated at a position 3c corresponding to a distal end 8a of the first sheath receiving portion 8, and the stress caused by separation is generated at the welding portion 12. Further, the stress caused by bending is dispersed at the first sheath receiving portion 8, and is diffused.

As described above, it is possible to move the positions at which the stress caused by bending and the stress caused by separation are generated, and it is possible to disperse the stress caused by bending and the stress caused by separation. Therefore, it is possible to prevent the first sheath 3 from bending deformation and rupturing at the position 3c corresponding to the proximal end 8a of the first sheath receiving portion 8, and to prevent the second sheath 4 from bending deformation and rupturing at the position 4c corresponding to the distal end 9a of the second sheath receiving portion 9.

Further, in the present embodiment, the first sheath 3 and the first sheath connecting portion 10 are connected by laser beam welding, and the second sheath 4 and the second sheath connecting portion 11 are also connected by laser beam welding. By this welding, it is possible to obtain high-strength and high-workability. On the other hand, since the laser beam welding follows heating, materials degenerate by heating in the welding portions 12 and 13 and the vicinity thereof, and accordingly the materials become fragile. However, since the stress caused by bending does not act on the welding portions 12 and 13 or the vicinity thereof, it is possible to obtain advantages using laser beam welding.

In addition, each of the step portions 10a and 11b is formed on the first sheath connecting portion 10 and the second sheath connecting portion 11. Thus, since each of the distal end 3a of the first sheath 3 and the proximal end 4b of the second sheath 4 contacts the step portions 10a and 11a, it is possible to engage the first sheath 3 with the first sheath receiving portion 8 reliably, and it is possible to engage the second sheath 4 with the second sheath receiving portion 9 reliably. In addition, since the step portions 10a, 11a are provided, an area in which the first sheath 3 and the first sheath connecting portion 10 are in contact and an area in which the second sheath 4 and the second sheath connecting portion 11 are in contact are expanded. As a result, it is possible to raise the connection-strength of the welding portions 12 and 13.

As described above, it is possible for the tubular member 6 to reliably connect the above two sheaths without any as bending deformation or rupturing. Accordingly, it is possible to extend an insertion portion of the endoscopic instrument, and to change the flexibility and the material of the sheath according to the type of operation. Further, when the two coil-sheaths are connected as in the present embodiment, if each of the coil-sheaths is multiplexed, and if the number of wire rods of one of the coil-sheaths differs from that of wire rods of the other of the coil-sheath, it is possible to connect the two coil-sheaths easily.

In this embodiment, two sheaths are connected to each other through the tubular member 6, however, this invention is not to be considered as limited by above. Three sheaths may be connected through a plurality of tubular members.

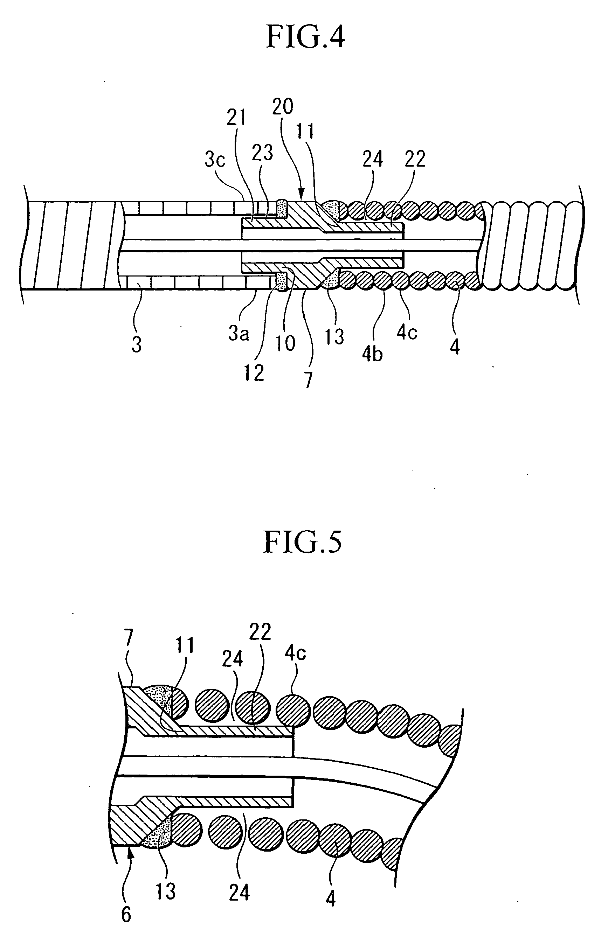

In FIG. 4 and FIG. 5, a first variant of the present embodiment is shown. As shown in FIG. 4, in a tubular member 20 of this variant, the external diameter of a first sheath receiving portion 21 is set so as to be provided with a gap 23 between the first sheath 3 and the first sheath receiving portion 21. Similarly, the external diameter of a second sheath receiving portion 22 is set so as to be provided with a gap 24 between the second sheath 4 and the second sheath receiving portion 22. In detail, the size of each of the gaps 23 and 24 is set so as to be within 0.2 millimeters.

As shown in FIG. 5, in the tubular member 20 of this variant, the gap 24 is deformed so as to become closer, for example, when the second sheath 4 is bent. Therefore, it is possible to disperse the stress caused by bending at the position 4c of the second sheath 4 in the axial direction of the second sheath receiving portion 22. Further, as shown in FIG 4, it is possible to disperse the stress caused by bending at the position 3c of the first sheath 3 in the axial direction of the first sheath receiving portion 21. Therefore, it is possible to obtain a high reliability relative to the bending deformation and the rupturing at the position 3c of the first sheath 3 and the position 4c of the second sheath 4.

### (Second embodiment)

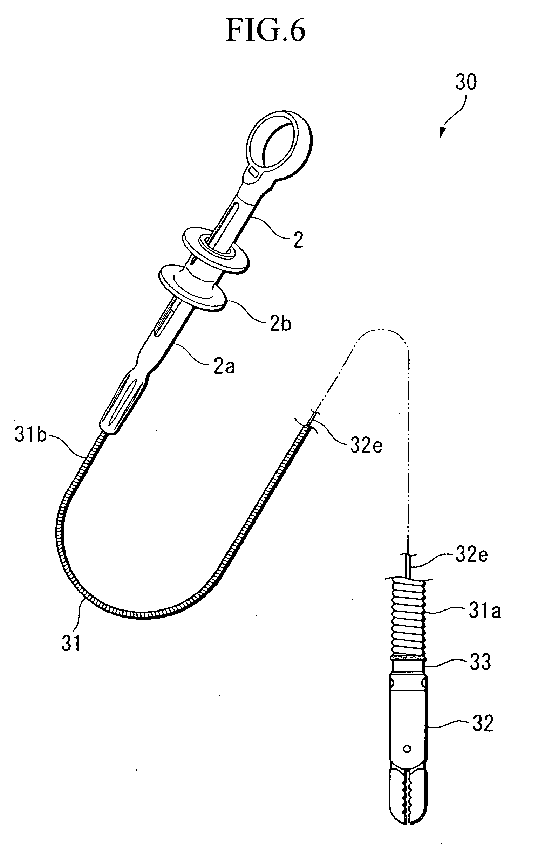

In FIG. 6, an endoscopic instrument of a second embodiment is shown. In this embodiment, the same components are given the same reference numerals used in the above embodiment, and an overlapping description thereof is omitted.

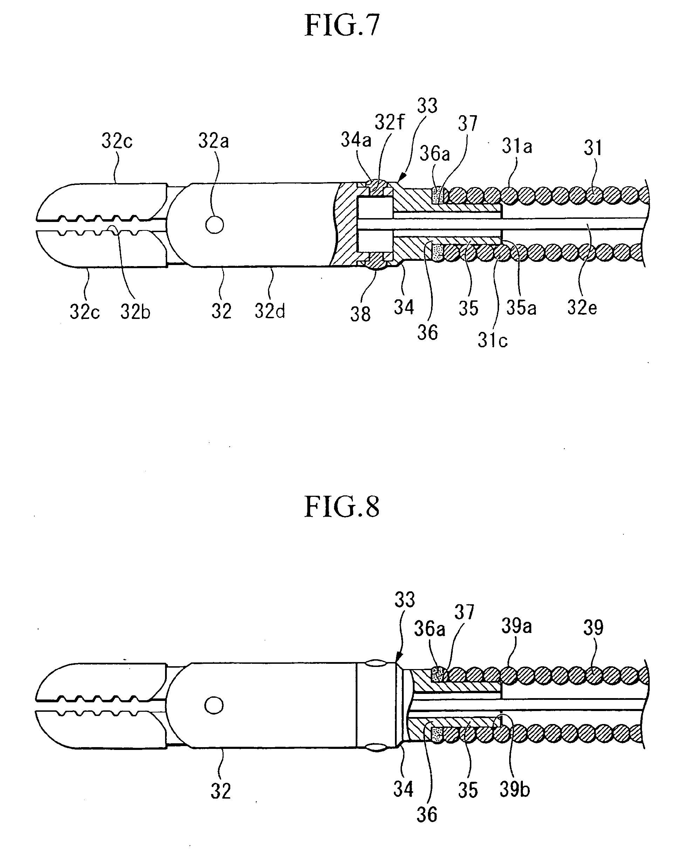

As shown in FIG 7, an endoscopic instrument 30 of this embodiment includes: a sheath 31 which is equivalent to the insertion portion extended from the operation portion 2; a treatment portion 32 which is disposed on a distal end 31a of the sheath 31; and a tubular member 33 which connects the sheath 31 with the treatment portion 32. The sheath 31 is made of metal, and is formed like a coil, and has flexibility.

As shown in FIG. 7, the treatment portion 32 is a biopsy forceps which includes: a pair of cups 32c which can be opened or closed around a shaft 32a as a center, and which is provided with a teeth portion 32c; a cover 32d which supports the shaft 32a; and an operating wire 32e which is connected to the pair of cups 32c. The pair of cups 32c opens by moving forward the operating wire 32e in an axial direction, and closes by moving backward the operating wire 32e in the axial direction. As shown in FIG. 6, the operating wire 32e is inserted into the sheath 31, and is connected to the slider 2b of the operation portion 2 which is disposed on a proximal end 31 b of the sheath 31. That is, when the slider 2b is moved forward on the holding portion 2a of the operation portion 2, the operating wire 32e moves forward, and when the slider 2b is moved backward on the holding portion 2a of the operation portion 2, the operating wire 32e moves backward. Therefore, it is possible to open or close the pair of cups 32c.

As shown in FIG. 7, the tubular member 33 is formed like a tube, and is made of metal. The tubular member 33 includes: a main body portion 34 which is disposed between the sheath 31 and the treatment portion 32 so that an axis of the main body portion 34 matches that of the distal end 31a of the sheath 31; a sheath receiving portion 35 which is disposed on a proximal end portion of the main body portion 34 and which is engaged inside of the sheath 31; and a sheath connecting portion 36 which is disposed on the main body portion 34 and the sheath receiving portion 35.

Further, a step portion 36a which contacts the distal end 31a of the sheath 31 engaged with the sheath receiving portion 35 is formed on the sheath connecting portion 36. The sheath connecting portion 36 and the sheath 31 which are made of metal are heated at the sheath connecting portion 36, and are connected by forming a welding portion 37 so as to weld the sheath connecting portion 36 and the sheath 31 in a circumferential direction using a laser beam.

A through hole 34e which penetrates the main body portion 34 from a side surface of the main body portion 34 in a radial direction is formed at a distal end of the main body portion 34. The cover 32d is inserted into a distal end of the tubular member 33, and has a through hole 32f which corresponds to the through hole 34a of the main body portion 34. A pin 38 is inserted into the through hole 34a of the main body portion 34 and the through hole 32f of the treatment portion 32. Therefore, the treatment portion 32 is fixed with the tubular member 33.

Next, the action of this embodiment will be explained.

As shown in FIG. 7, the sheath 31is fixed with the sheath connecting portion 36 by the welding portion 37 so as to prevent the sheath 31 from separating from the sheath connecting portion 36 in an axial direction. Further, the sheath 31 is supported in a radial direction so as to oppose bending by the sheath receiving portion 35 of the tubular member 33. which is closer to a proximal end of the tubular member 33 than the welding portion 37.

When the sheath 31 is bent, an axial force which causes separation is generated, and a bending action occurs, between the tubular member 33 and the sheath 31. Therefore, a stress caused by bending and a stress caused by separation are generated in the sheath 3. Since the sheath 31 is supported in the radial direction by the sheath receiving portion 35, the stress caused by bending is generated at a position 31c corresponding to a proximal end 35a of the sheath receiving portion 35 of the tubular member 33. On the other hand, the stress caused by separation is generated at the welding portion 37 which fixes the distal proximal end 31a of the sheath 31. At this time, the sheath 31 is not restricted, and is free in an axial direction. Therefore, the sheath 31 being supported by the sheath receiving portion 35 is extended by the stress. As a result, the stress caused by bending is dispersed, and is diffused.

As described above, a position at which the stress caused by bending is generated differs from a position at which the stress caused by separation is generated, thus the stress caused by bending and the stress caused by separation are dispersed. Therefore, it is possible to prevent the sheath 31 from bending deformation and rupturing at the position 31c of the sheath 31 corresponding to the distal end 35a of the sheath receiving portion 35. Since the stress caused by bending does not act on the welding portion 37 and the vicinity thereof, it is possible to obtain advantages using laser beam welding (that is, high-strength and high-workability). Further, since the step portion 36a is formed on the sheath connecting portion 36, it is possible to reliably engage the sheath 31 with the sheath receiving portion 35 by contacting the sheath 31 and the step portion 36a. Furthermore, since an area in which the sheath 31 and the sheath connecting portion 36 are in contact is expanded, it is possible to raise the connection strength of the welding portion 37.

As described above, it is possible for the tubular member 33 to reliably connect the sheath 31 and the treatment device 32 without such bending deformation or rupturing. Note that, an example where the biopsy forceps is used as the treatment device 32 is given, however, this present invention is not limited to this. It is possible to use a kind of forceps such as a holding forceps and a clipping forceps instead of the biopsy forceps. Further, it is possible to reliably connect various treatment portions such as a high-frequency snare and a clip, using the tubular member 33.

In FIG. 8, a first variant of the present embodiment is shown. As shown in FIG. 8, in this variant, a case in which an internal diameter of a sheath 39 is smaller than an external diameter of the sheath receiving portion 35 of the tubular member 33 is shown. A circumferential face of the sheath 39 is ground at a distal end 39a of the sheath 39, and a diameter of the distal end 39a is set so as to engage the distal end 39a with the sheath receiving portion 35. As to this, it is preferable to process the distal end 39a of the sheath 39 so as to engage the tubular member with the sheath.

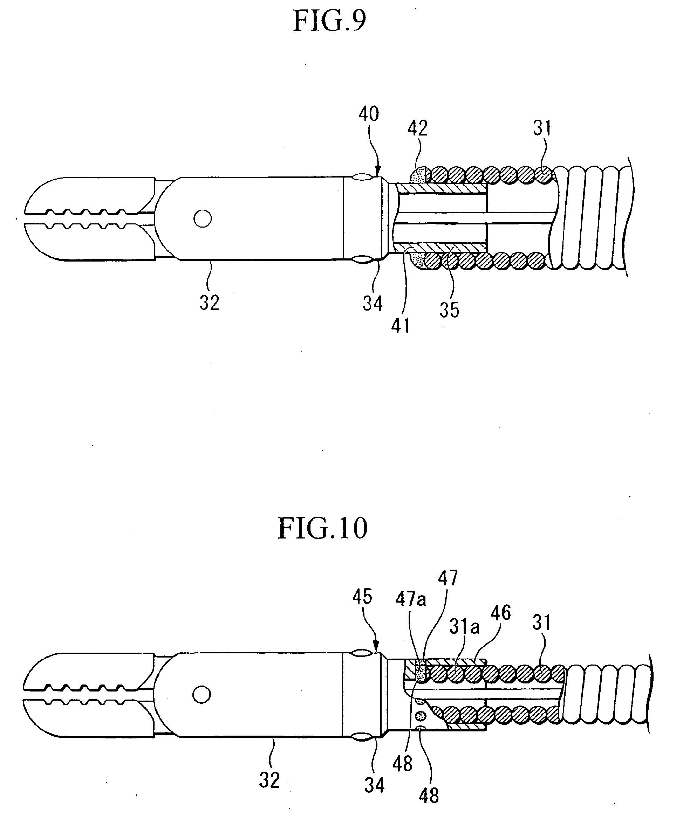

In FIG. 9, a second variant of the present embodiment is shown. As shown in FIG. 9, in this variant, there is not a step portion on a sheath connecting portion 41 of a tubular member 40. However, in the tubular member 40, a welding portion 42 is formed on the sheath connecting portion 41. Therefore, advantages as described above can be obtained.

In FIG. 10, a third variant of the present embodiment is shown. As shown in FIG. 10, in this variant, an internal diameter of a sheath receiving portion 46 of a tubular member 45 is substantially equal with the external diameter of the first sheath 31, and is set so as to be removably attached in axial direction with ease. Further, a step portion 47a of a sheath connecting portion 47 is formed inside. Therefore, the sheath receiving portion 46 is engaged with the outside of the sheath 31, and the distal end 31a of the sheath 31 contacts the step portion 47a. Further, in the sheath connecting portion 47, the distal end 31a of the sheath 31 is discontinuously connected to the sheath receiving portion 46 at a plurality of locations in a circumferential direction, by laser-welding outside of the sheath connecting portion 47. Therefore, a welding portion 48 is formed.

As described above, since the sheath receiving portion 46 of the tubular member 45 is engaged outside of the sheath 31, advantages as described above can be obtained. Further, in this case, the welding portion 48 between the sheath 31 and the sheath connecting portion 47 of the tubular member 45 is inside of the sheath connecting portion 47, however, both can be connected to each other by laser-welding outside of the sheath connecting portion 47. Further, similarly to this variant, if the welding portion 48 has strength in the axial direction, the sheath 31 and the sheath connecting portion 47 may be discontinuously connected to each other in a circumferential direction, but continuously connecting.

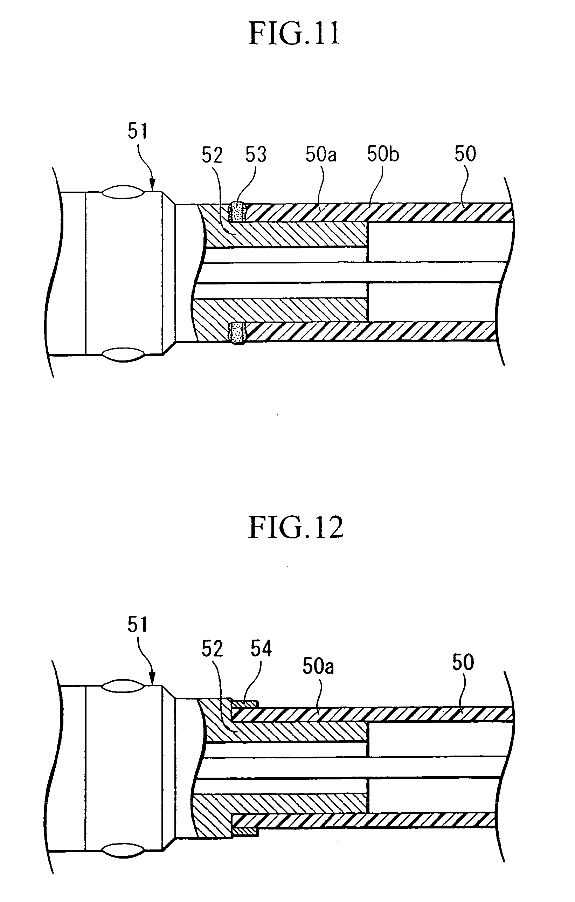

In FIG. 11, a fourth variant of the present embodiment is shown. As shown in FIG. 11, in this variant, a sheath 50 and tubular member 51 are made of resin. A distal end 50a of the sheath 50 and a sheath connecting portion 52 are connected to each other through a welding portion 53 which is formed by ultrasonic welding. As described above, if the sheath 50 is made of resin, the tubular member 51 is also made of resin. Thus the sheath 50 and the tubular member 51 may be fixed to each other by welding. Further, when the sheath 50 is bent, a position 50b at which a stress caused by bending is generated may shift relative to the welding portion 53 in the axial direction. Therefore, the advantages as described above can be obtained.

In FIG 12, a fifth variant of the present embodiment is shown. As shown in FIG. 12, in this variant, the distal end 50a of the sheath 50 is engaged outside of the sheath connecting portion 52 of the tubular member 51, and a ring 54 which is made of metal is engaged outside of the distal end 50a of the sheath 50. A rod is inserted inside of the sheath 50, then the ring 54 is pressed toward the center of the sheath 50 from outside thereof, and plastically deformed. Therefore, the distal end 50a of the sheath 50 is fixed to the sheath connecting portion 52. According to this fixing method, the advantages as described above can be obtained.

The contents of the embodiments as described above are not to be considered as limiting to each of embodiments, and they may be combined suitably. In each of the embodiments, connecting methods for connecting the distal end of the sheath to the sheath connecting portion of the tubular member are explained. However, this invention is not to be considered as limited to these connecting methods. The sheath and the tubular member may be fixed to each other at least a limited area of the sheath connecting portion, while predetermined strength is obtained.

## Claims

1. A tubular member (6, 20, 33, 40, 45, 51) connected to an end (3a, 4a, 31a, 39a, 50a)of a flexible sheath (3, 4, 31, 39, 50), comprising:
a main body portion (7, 34); and
a sheath receiving portion (8, 9, 21, 22, 35, 46) disposed on at least one end of the main body portion (7, 34), and engaged with the inside or the outside of the sheath (3, 4, 31, 39, 50), thereby supporting the sheath (3, 4, 31, 39, 50) in a radial direction;
**characterized in that** a sheath connecting portion (10, 11, 36, 41, 47, 52) is disposed between the main body portion (7, 34) and the sheath receiving portion (8, 9, 21, 22, 35, 46), wherein the sheath connecting portion (10, 11, 36, 41, 47, 52) is fixed to the end (3a, 4a, 31a, 39a, 50a) of the sheath (3, 4, 31, 39, 50) so as to prevent the sheath (3, 4, 31, 39, 50) from separating from the sheath connecting portion (10, 11, 36, 41, 47, 52) in an axial direction,
wherein the sheath (3, 4, 31, 39, 50) is free to extend in axial direction along the sheath receiving portion (18, 9, 21, 22, 23, 46).

2. The tubular member (6, 20, 33, 40, 45, 51) according to claim 1, wherein an external diameter or an internal diameter of the sheath receiving portion (8, 9, 21, 22, 35, 46) is set so as to provide a gap (23, 24) between the sheath receiving portion (8, 9, 21, 22, 35, 46) and the sheath (3, 4, 31, 39, 50).

3. The tubular member (6, 20, 33, 40, 45, 51) according to claim 1, wherein the sheath connecting portion (10, 11, 36, 41, 47, 52) comprises a step portion (10a, 11a, 36a, 47a) which contacts the end (3a, 4a, 31a, 39a, 50a) of the sheath (3, 4, 31, 39, 50).

4. The tubular member (6, 20, 33, 40, 45, 51) according to claim 1, wherein the sheath connecting portion (10, 11, 36, 41, 47, 52) and the sheath (3, 4, 31, 39, 50) are made of metal, thus the sheath connecting portion (10, 11, 36, 41, 47, 52) is capable being fixed to the sheath (3, 4, 31, 39, 50) by heating.

5. The tubular member (6, 20, 33, 40, 45, 51) according to claim 3, wherein the sheath (3, 4, 31, 39, 50) is made of metal and formed like a coil.

6. An endoscopic instrument (1, 30), comprising:
the tubular member (6, 20, 33, 40, 45, 51) of any preceding claim, wherein the sheath connecting portion (10, 11, 36, 41, 47, 52) is disposed on a proximal end of the main body portion (7, 34) and is fixed to a distal end (3a, 31a, 39a, 50a) of the sheath (3, 4, 31, 39, 50);
wherein the sheath (3, 4, 31, 39, 50) is inserted into a channel of the endoscope; and
a treatment portion (5, 32) connected to a distal end of the main body portion (7, 34) of the tubular member (6, 20, 33, 40, 45, 51).

7. An endoscopic instrument (1, 30), comprising:
the tubular member (6, 20, 33, 40, 45, 51) of claims 1-5 further comprising first and second sheath receiving portions (8, 9, 21, 22, 35, 46) disposed on opposing ends of the main body portion (7, 34), wherein the main body portion (7, 34) is disposed between the first and second sheaths; and
wherein the sheath (3, 4, 31, 39, 50) is inserted into a channel of the endoscope.

## Patentansprüche

1. Rohrförmiges Element (6, 20, 33, 40, 45, 51), das mit einem Ende (3a, 4a, 31a, 39a, 50a) eines flexiblen Mantels (3, 4, 31, 39, 50) verbunden ist, aufweisend:
einen Hauptkörperabschnitt (7, 34); und
einen Mantelaufnahmeabschnitt (8, 9, 21, 22, 35, 46), der an mindestens einem Ende des Hauptkörperabschnitts (7, 34) angeordnet und mit der Innenseite oder der Außenseite des Mantels (3, 4, 31, 39, 50) in Eingriff steht und **dadurch** den Mantel (3, 4, 31, 39, 50) in radialer Richtung stützt;
**dadurch gekennzeichnet, dass** ein Mantelverbindungsabschnitt (10, 11, 36, 41, 47, 52) zwischen dem Hauptkörperabschnitt (7, 34) und dem Mantelaufnahmeabschnitt (8, 9, 21, 22, 35, 46) angeordnet ist, wobei der Mantelverbindungsabschnitt (10, 11, 36, 41, 47, 52) am Ende (3a, 4a, 31a, 39a, 50a) des Mantels (3, 4, 31, 39, 50) so befestigt ist, dass der Mantel (3, 4, 31, 39, 50) daran gehindert wird, sich in axialer Richtung vom Mantelverbindungsabschnitt (10, 11, 36, 41, 47, 52) zu trennen, wobei der Mantel (3, 4, 31, 39, 50) sich frei in axialer Richtung entlang dem Mantelaufnahmeabschnitt (8, 9, 21, 22, 35, 46) ausdehnen kann.

2. Rohrförmiges Element (6, 20, 33, 40, 45, 51) nach Anspruch 1, bei dem der Außendurchmesser oder der Innendurchmesser des Mantelaufnahmeabschnitts (8, 9, 21, 22, 35, 46) so eingestellt ist, dass ein Spalt (23, 24) zwischen dem Mantelaufnahmeabschnitt (8, 9, 21, 22, 35, 46) und dem Mantel (3, 4, 31, 39, 50) gebildet wird.

3. Rohrförmiges Element (6, 20, 33, 40, 45, 51) nach Anspruch 1, bei dem der Mantelverbindungsabschnitt (10, 11, 36, 41, 47, 52) einen Stufenabschnitt (10a, 11a, 36a, 47a) aufweist, der das Ende (3a, 4a, 31a, 39a, 50a) des Mantels (3, 4, 31, 39, 50) berührt.

4. Rohrförmiges Element (6, 20, 33, 40, 45, 51) nach Anspruch 1, bei dem der Mantelverbindungsabschnitt (10, 11, 36, 41, 47, 52) und der Mantel (3, 4, 31, 39, 50) aus Metall bestehen, so dass der Mantelverbindungsabschnitt (10, 11, 36, 41, 47, 52) durch Erhitzen am Mantel (3, 4, 31, 39, 50) befestigt werden kann.

5. Rohrförmiges Element (6, 20, 33, 40, 45, 51) nach Anspruch 3, bei dem der Mantel (3, 4, 31, 39, 50) aus Metall besteht und wie eine Spule geformt ist.

6. Endoskopisches Instrument (1, 30), aufweisend:
das rohrförmige Element (6, 20, 33, 40, 45, 51) nach einem der vorigen Ansprüche, wobei der Mantelverbindungsabschnitt (10, 11, 36, 41, 47, 52) am proximalen Ende des Hauptkörperabschnitts (7, 34) angeordnet und am distalen Ende (3a, 31a, 39a, 50a) des Mantels (3, 4, 31, 39, 50) befestigt ist;
wobei der Mantel (3, 4, 31, 39, 50) in einen Kanal des Endoskops eingeführt ist; und
ein Behandlungsabschnitt (5, 32) mit dem distalen Ende des Hauptkörperabschnitts (7, 34) des rohrförmigen Elements (6, 20, 33, 40, 45, 51) verbunden ist.

7. Endoskopisches Instrument (1, 30), aufweisend:
das rohrförmige Element (6, 20, 33, 40, 45, 51) nach Anspruch 1 bis 5, ferner einen ersten und zweiten Mantelaufnahmeabschnitt (8, 9, 21, 22, 35, 46) aufweisend, die an gegenüberliegenden Enden des Hauptkörperabschnitts (7, 34) angeordnet sind, wobei der Hauptkörperabschnitt (7, 34) zwischen dem ersten und zweiten Mantel angeordnet ist; und
wobei der Mantel (3, 4, 31, 39, 50) in einen Kanal des Endoskops eingeführt ist.

## Revendications

1. Élément tubulaire (6, 20, 33, 40, 45, 51) connecté à une extrémité (3a, 4a, 31a, 39a, 50a) d'une gaine flexible (3, 4, 31, 39, 50), comprenant :
une partie de corps principale (7, 34) ; et
une partie de réception de gaine (8, 9, 21, 22, 35, 46) disposée sur au moins une extrémité de la partie de corps principale (7, 34) et engagée avec l'intérieur ou l'extérieur de la gaine (3, 4, 31, 39, 50), supportant ainsi la gaine (3, 4, 31, 39, 50) dans une direction radiale ;
**caractérisé en ce qu'**une partie de connexion de gaine (10, 11, 36, 41, 47, 52) est disposée entre la partie de corps principale (7, 34) et la partie de réception de gaine (8, 9, 21, 22, 35, 46), dans lequel la partie de connexion de gaine (10, 11, 36, 41, 47, 52) est fixée à l'extrémité (3a, 4a, 31a, 39a, 50a) de la gaine (3, 4, 31, 39, 50) de façon à empêcher que la gaine (3, 4, 31, 39, 50) ne se sépare de la partie de connexion de gaine (10, 11, 36, 41, 47, 52) dans une direction axiale,
dans lequel la gaine (3, 4, 31, 39, 50) est libre de s'étendre dans la direction axiale le long de la partie de réception de gaine (18, 9, 21, 22, 23, 46).

2. Élément tubulaire (6, 20, 33, 40, 45, 51) selon la revendication 1, dans lequel un diamètre externe ou un diamètre interne de la partie de réception de gaine (8, 9, 21, 22, 35, 46) est fixé de façon à créer un espace (23, 24) entre la partie de réception de gaine (8, 9, 21, 22, 35, 46) et la gaine (3, 4, 31, 39, 50).

3. Élément tubulaire (6, 20, 33, 40, 45, 51) selon la revendication 1, dans lequel la partie de connexion de gaine (10, 11, 36, 41, 47, 52) comprend une partie en marche (10a, 11a, 36a, 47a) qui est au contact de l'extrémité (3a, 4a, 31a, 39a, 50a) de la gaine (3, 4, 31, 39, 50).

4. Élément tubulaire (6, 20, 33, 40, 45, 51) selon la revendication 1, dans lequel la partie de connexion de gaine (10, 11, 36, 41, 47, 52) et la gaine (3, 4, 31, 39, 50) sont constituées d'un métal, ainsi la partie de connexion de gaine (10, 11, 36, 41, 47, 52) est apte à être fixée à la gaine (3, 4, 31, 39, 50) par chauffage.

5. Élément tubulaire (6, 20, 33, 40, 45, 51) selon la revendication 3, dans lequel la gaine (3, 4, 31, 39, 50) est constituée d'un métal et formée comme un enroulement.

6. Instrument endoscopique (1, 30), comprenant :
l'élément tubulaire (6, 20, 33, 40, 45, 51) selon une quelconque revendication précédente, dans lequel la partie de connexion de gaine (10, 11, 36, 41, 47, 52) est disposée sur une extrémité proximale de la partie de corps principale (7, 34) et est fixée à une extrémité distale (3a, 31a, 39a, 50a) de la gaine (3, 4, 31, 39, 50) ;
dans lequel la gaine (3, 4, 31, 39, 50) est insérée dans un canal de l'endoscope ; et
une partie de traitement (5, 32) connectée à une extrémité distale de la partie de corps principale (7, 34) de l'élément tubulaire (6, 20, 33, 40, 45, 51).

7. Instrument endoscopique (1, 30), comprenant :
l'élément tubulaire (6, 20, 33, 40, 45, 51) selon les revendications 1 à 5 comprenant en outre des parties de réception (8, 9, 21, 22, 35, 46) de première et deuxième gaines disposées sur des extrémités opposées de la partie de corps principale (7, 34), dans lequel la partie de corps principale (7, 34) est disposée entre la première et la deuxième gaines ; et
dans lequel la gaine (3, 4, 31, 39, 50) est insérée dans un canal de l'endoscope.
